# EUROPÄISCHE PATENTSCHRIFT

(11) **EP 0 575 364 B1**
(45) Veröffentlichungstag und Bekanntmachung des Hinweises auf die Patenterteilung: **24.04.1996**
(21) Anmeldenummer: 92905405.4
(22) Anmeldetag: 26.02.1992
(51) Int. Cl.: G01N 33/52

(54) **TESTTRÄGER ZUR BESTIMMUNG EINES ANALYTEN AUS VOLLBLUT**
TEST CARRIER FOR DETERMINING AN ANALYTE IN WHOLE BLOOD
SUPPORT DE TEST POUR LA DETERMINATION D'UN ECHANTILLON DE SANG TOTAL A ANALYSER

(30) Priorität: 28.02.1991 DE 4106293
(43) Veröffentlichungstag der Anmeldung: 29.12.1993
(73) Patentinhaber: BOEHRINGER MANNHEIM GMBH, D-68298 Mannheim (DE)
(72) Erfinder: FREY, Günter, D-6701 Ellerstadt (DE); HORN, Doris, D-6834 Ketsch (DE)
(74) Vertreter: Pfeifer, Hans-Peter, Dr. Patentanwalt
(86) Internationale Anmeldenummer: DE9200154
(87) Internationale Veröffentlichungsnummer: WO9215879

(56) Entgegenhaltungen:
- EP-A- 0 256 806
- EP-A- 0 274 911
- EP-A- 0 302 287

## Beschreibung

Die Erfindung betrifft ein Testfeld für einen Testträger zur Bestimmung eines Analyten aus Vollblut mit Hilfe eines in dem Testträger enthaltenen Reagenzsystems, welches ein Farbbildungsreagenz einschließt, mit einem Testfeld, welches eine Probenaufgabeseite, der die Blutprobe zugeführt wird und eine Nachweisseite, auf der infolge der Reaktion des Analyten mit dem Reagenzsystem eine optisch nachweisbare Veränderung stattfindet, aufweist, wobei das Testfeld so ausgebildet ist, daß in der Probe enthaltende Erythrozyten nicht auf die Nachweisseite gelangen.

Zur qualitativen oder quantitativen analytischen Bestimmung von Bestandteilen von Blut werden zunehmend sogenannte trägergebundene Tests verwendet. Bei diesen ist ein Reagenzsystem in mindestens einem aus einer oder mehreren Schichten bestehenden Testfeld eines festen Testträgers eingebettet, das mit der Probe in Kontakt gebracht wird. Die Reaktion von Probe und Reagenzsystem führt zu einer optisch nachweisbaren Veränderung, insbesondere einem Farbumschlag, welcher visuell oder mit Hilfe eines Gerätes, meist reflexionsphotometrisch ausgewertet werden kann. Statt zu einem Farbumschlag kann die Reaktion auch zum Entstehen oder zur Veränderung eines anderen optisch nachweisbaren Signals führen, beispielsweise eine Fluoreszenz oder einer Lumineszenz.

Testträger (welche in der angelsächsischen Literatur vielfach auch als "solid reagent analysis elements" bezeichnet werden) sind häufig als Teststreifen ausgebildet, die im wesentlichen aus einer länglichen Tragschicht aus Kunststoffmaterial und einem oder mehreren darauf angebrachten Testfeldern bestehen. Es sind jedoch auch Testträger bekannt, bei denen ein Testfeld von einem Rahmen aus Kunststoff ähnlich einem Diapositiv umgeben ist.

Trägergebundene Tests zeichnen sich insbesondere durch ihre einfache Handhabung aus. Um so bedauerlicher ist es, daß bei den meisten bisher bekannten Testträgern das Blut nicht unmittelbar als Vollblut verwendet werden kann. Es ist vielmehr erforderlich, die roten Blutkörperchen (Erythrozyten) abzutrennen, um farbloses Plasma bzw. Serum zu gewinnen. Dies geschieht üblicherweise durch Zentrifugieren. Damit ist jedoch ein zusätzlicher Handhabungsschritt verbunden, der eine verhältnismäßig große Blutmenge und aufwendige Apparatur erfordert.

Es hat daher nicht an Versuchen gefehlt, Testträger zur Verfügung zu stellen, welche analytische Bestimmungen unmittelbar aus Blut ermöglichen. Dabei kann man zwei grundsätzlich verschiedene Lösungsmöglichkeiten unterscheiden.

Bei dem ersten Lösungsansatz erfolgt die visuelle oder apparative Auswertung des Farbumschlags auf der gleichen Seite des Testfeldes, auf die auch die Probe aufgegeben wird. Dabei ist das Testfeld so aufgebaut, daß der Analyt aus der Probe durch die Testfeldoberfläche zu den Reagenzien gelangt, während die Erythrozyten zurückgehalten werden. Nach einer definierten Zeit wird die Blutprobe von der Testfeldoberfläche abgewischt oder abgewaschen und der Farbumschlag beobachtet. Beispiele solcher Testträger sind in der US-A-3 630 957 und in der EP-B-130 335 und der EP-A-0 217 246 beschrieben.

Bei dem zweiten Lösungsansatz wird die Probe auf der einen Seite des Testfeldes aufgegeben (Probenaufgabeseite) und der Farbumschlag auf der anderen Seite (Nachweisseite) registriert. Ein wesentlicher Vorteil dieser Verfahrensweise besteht darin, daß das Blut nicht abgewischt oder abgewaschen werden muß. Man bezeichnet solche Testträger deswegen auch als nicht abzuwischende Testträger (non wipe test carriers).

Mit dem Abwischen entfällt nicht nur ein lästiger Handhabungsschritt, sondern auch eine mögliche Fehlerquelle, die daraus resultieren kann, daß der Zeitpunkt, zu dem Blut entfernt werden muß, nicht genau eingehalten wird. Andererseits ist dieser Lösungsansatz besonders schwer zu realisieren. Erforderlich ist ein Erythrozyten- bzw. Blutfarbstoffilter, der einerseits die intensiv färbenden Bestandteile des Blutes zuverlässig zurückhält, andererseits den Analyt vollständig und ausreichend schnell passieren läßt. Es erweist sich als außerordentlich schwierig, einen Testfeldaufbau zu finden, der diese Anforderungen erfüllt.

In der US-A-36 63 374 und in der US-A-42 56 693 wird ein Membranfilter eingesetzt. Zwar sind Membranfilter für das Abfiltern von Erythrozyten grundsätzlich geeignet, ihre Verwendung in Testträgern hat sich jedoch nicht bewährt. Das gleiche gilt für die ebenfalls in diesen US-Patenten erwähnte Kombination des Membranfilters mit einer Glasfaserschicht, welche das Verstopfen des Membranfilters mit gröberen Teilchen verhindern soll. Die Herstellung derartiger Testträger wäre sehr aufwendig, ohne daß eine befriedigende Funktion erreicht wird.

In der US-A-4 069 017 und mehreren US-Patenten der gleichen Anmelderin wird ebenfalls die Möglichkeit angesprochen, in einem Testträger eine den Durchtritt von Erythrozyten verhindernde Zwischenschicht vorzusehen, welche zugleich strahlenblockierende Bestandteile enthält, um sicherzustellen, daß die Lichtstrahlen des Auswertegerätes nicht in die erythrozytenhaltige Schicht vordringen können. In dieser Schrift sind jedoch keine Angaben enthalten, wie die Zwischenschicht im einzelnen aufgebaut sein könnte, um die Filterung der Erythrozyten zu erreichen.

Die US-A-4 824 639 beschreibt die Verwendung einer asymmetrischen Membran, wie sie für technische Trennverfahren (umgekehrte Osmose) bekannt ist, für die Zwecke der Erythrozytenabtrennung. Die Membran wird in einem Koagulationsverfahren durch Eintauchen einer teilweise oder vollständig gelierten Schicht in ein Koagulationsbad hergestellt und kann auch Reagenzien enthalten. Dabei soll die optische Auswertung sowohl von der Blutaufgabeseite her (nach Abwischen) als auch von der gegenüberliegenden Seite her möglich sein.

In der EP-A-0 302 287 ist ein Testfeld dargestellt, welches einen Schichtverbund aufweist, der durch Flüssigbeschichten einer das Farbbildungsreagenz enthaltenden Nachweisschicht auf eine der Probenaufgabeseite zugewandte Basisschicht hergestellt ist. Die Basisschicht enthält einen polymeren Filmbildner, Kieselgur und ein Pigment. Die Nachweisschicht enthält einen polymeren Filmbildner, welcher beim Flüssigbeschichten teilweise in die Basisschicht eindringt und eine Übergangszone bildet, in der die Erythrozyten zurückgehalten werden.

Das durch die Farbstoff-Bestandteile des Blutes verursachte Problem wird noch zusätzlich dadurch verschärft, daß das üblicherweise verwendete, durch Einstich in die Haut gewonnene Kapillarblut wegen der damit verbundenen mechanischen Beanspruchung unvermeidlich teilweise hämolysiert ist, d.h. es enthält aus zerstörten Erythrozyten frei gewordenen Blutfarbstoff. Obwohl der Hömolyseanteil im allgemeinen unter 0,1 % liegt, verursacht der freie Blutfarbstoff wegen seiner intensiven Farbe selbst dann noch Meßungenauigkeiten, wenn die Erythrozyten vollständig abgetrennt wurden.

Da keine der vorbekannten Realisierungen eines NW-Testträgers mit Erythrozytenabtrennung in jeder Hinsicht befriedigende Eigenschaften aufweist, wurde bei einer im Handel befindlichen Realisierung eines solchen Testträgers auf die Abtrennung der Erythrozyten vollständig verzichtet und die Störung durch die intensive Rotfärbung meßtechnisch mit Hilfe einer Messung bei zwei verschiedenen Wellenlängen kompensiert. Dies beeinträchtigt jedoch die Meßgenauigkeit, weil die dunkle Blutfarbe den Signalhub, d.h. die Differenz der gemessenen diffusen Reflexion innerhalb des Meßbereiches des Tests wesentlich (etwa auf die Hälfte) vermindert. Außerdem wird der Aufwand für die apparative Auswertung stark erhöht und es ist keine visuelle Kontrolle des Farbumschlags möglich.

Der Erfindung liegt die Aufgabe zugrunde, ein Testfeld für einen NW-Testträger zur Verfügung zu stellen, welches eine zuverlässige Abtrennung des Blutfarbstoffs und eine schnelle und intensive Bildung des optischen Nachweissignals (und demzufolge eine hohe Meßgenauigkeit) ermöglicht. Zugleich soll es möglichst einfach herzustellen sein.

Die Aufgabe wird bei einem Testfeld der eingangs bezeichneten Art dadurch gelöst, daß es eine kombinierte Blutfarbstoffabtrenn- und Nachweisschicht aufweist, welche aus einer Dispersion oder Emulsion eines polymeren Filmbildners gebildet ist, die in homogener Verteilung ein Pigment in einer Konzentration von mindestens 30 Gew.-%, bezogen auf den Feststoffgehalt der filmbildenden Masse, den polymeren Filmbildner, das Farbbildungsreagenz und ein Quellmittel enthält, wobei die Quellfähigkeit des Quellmittels so hoch ist, daß die optisch nachweisbare Veränderung auf der Nachweisseite nach maximal einer Minute meßbar ist.

Dispersionsfilmbildner enthalten mikroskopische, in der Trägerflüssigkeit (meist Wasser) unlösliche Polymerteilchen, welche in feinster Verteilung in der Trägerflüssigkeit dispergiert sind. Wird bei der Filmbildung die Flüssigkeit durch Verdampfen entfernt, so nähern sich die Teilchen und berühren sich schließlich. Durch die dabei auftretenden großen Kräfte und einen mit der Filmbildung einhergehenden Gewinn an Oberflächenenergie wachsen die Teilchen zu einer weitgehend geschlossenen Filmschicht zusammen. Nähere Einzelheiten hierzu sind beispielsweise dem Artikel "Latex film formation" von J.W. Vanderhoff in Polymer News 1977, Seite 194 bis 203, zu entnehmen.

Alternativ kann auch eine Emulsion des Filmbildners verwendet werden, bei der dieser in einem Lösungsmittel gelöst ist. Das gelöste Polymer ist in einer Trägerflüssigkeit emulgiert, die mit dem Lösungsmittel nicht mischbar ist.

Als Polymere für solche Filmbildner eignen sich insbesondere Polyvinylester, Polyvinylacetate, Polyacrylester, Polymethylacrylsäure, Polyvinylamide, Polyamide und Polystyrol. Neben Homopolymeren sind auch Mischpolymerisate z.B. von Butadien, Styrol oder Maleinsäureester geeignet.

Der mittlere Partikeldurchmesser der Pigmentpartikel liegt vorzugsweise zwischen 0,2 und 1µm. Titandioxid ist ein weit verbreitetes und auch für die Erfindung besonders geeignetes Pigment.

Die Eigenschaften der Blutfarbstoffabtrenn- und Nachweisschicht hängen selbstverständlich nicht nur von dem Filmbildner, dem Pigment und dem Quellmittel ab, sondern auch von den in dieser Schicht enthaltenen Bestandteilen der Testrezeptur. Diese umfaßt neben dem eigentlichen Reagenzsystem meist auch Hilfsstoffe, wie zum Beispiel Detergentien, Puffer und Schutzkolloide.

Infolge dieser Vielfalt möglicher Varianten ist es nicht möglich, universelle Regeln anzugeben, wie die schichtbildende Masse, aus der die Blutfarbstoffabtrenn- und Nachweisschicht gebildet wird, quantitativ zusammengesetzt sein soll. Der Fachmann kann auf Basis der hier gegebenen Informationen die Eignung einer gewählten Zusammensetzung jedoch ohne weiteres testen und die Rezeptur der schichtbildenden Masse gezielt optimieren.

Der Pigmentanteil liegt mit mindestens 30 Gew-%, bevorzugt mindestens 40 Gew-%, bezogen auf den gesamten Feststoffgehalt der schichtbildenden Masse ungewöhnlich hoch. Dadurch ergeben sich selbst bei einer extrem dünnen Schichtdicke der Blutfarbstoffabtrenn- und Nachweisschicht gute strahlungsblockierende Eigenschaften, d.h. die rote Farbe auf der Probenaufgabeseite stört die optische Auswertung nicht.

Überraschenderweise wurde gefunden, daß man durch Zugabe eines gut quellenden Quellmittels (d.h. einer Substanz, die unter Aufnahme von Wasser ihr Volumen vergrößert) nicht nur eine Schicht erhält, die relativ schnell von der Probenflüssigkeit penetriert wird, sondern trotz dieser Öffnungswirkung des Quellmittels gute Blutfarbstoffabtrenneigenschaften resultieren. Die erforderliche Qualität und Menge des Quellmittels läßt sich auf Basis der hier gegebenen Erläuterungen empirisch für die jeweilige Schichtzusammensetzung festlegen. Die Quelleigenschaften sollten jedenfalls mindestens so gut sein, daß für einen Test, bei dem die Geschwindigkeit der Farbbildung - wie beispielsweise bei der üblichen Glucosenachweisreaktion - überwiegend von der Penetration der Probenflüssigkeit durch die Schicht abhängt, die optisch nachweisbare Reaktion nach maximal einer Minute meßbar ist.

Als besonders geeignetes Quellmittel hat sich Methylvinylethermaleinsäureanhydrid-Copolymer erwiesen.

Aus den im Zusammenhang mit der vorliegenden Erfindung durchgeführten Experimenten läßt sich ableiten, daß Filmbildner und Pigment in einem annähernd gleichen Gewichtsverhältnis zueinander stehen sollten. Je nach den Umständen des Einzelfalls erweisen sich Gewichtsrelationen des Filmbildners zu dem Pigment zwischen 1:10 und 2:1 als bevorzugt.

Im Regelfall ist es vorteilhaft, wenn der Gesamtanteil der Summe von Filmbildner und Pigment am Feststoffgehalt der schichtbildenden Masse relativ hoch (meist über 70%) ist. Im Zusammenhang mit Rezepturen, die wegen Besonderheiten der Analysereaktion einen ungewöhnlich hohen Gewichtsanteil des Reagenzsystems erfordern, wurde jedoch auch mit einem Gesamtfeststoffgehalt von Filmbildner und Pigment von 40%, bezogen auf den Feststoffgehalt der filmbildenden Masse, positive Ergebnisse erzielt. Inerte anorganische Partikel mit einem mittleren Partikeldurchmesser von erheblich mehr als 1 µm, die keine oder schlechte Pigmenteigenschaften haben, wie beispielsweise Kieselgur (Diatomeenerde) sollten nicht oder nur in einer sehr geringen Konzentration von weniger als 10 % in der filmbildenden Masse enthalten sein.

Die Herstellung der kombinierten Blutfarbstoffabtrennund Nachweisschicht aus einer homogenen Dispersion der genannten Bestandteile führt zu einer praktisch homogenen Schichtstruktur. Hierdurch unterscheidet sich die erfindungsgemäße Blutfarbstoffabtrenn- und Nachweisschicht grundsätzlich von der asymmetrischen Membran gemäß der eingangs erwähnten US-A-4 824 639.

Wesentlich ist, daß die kombinierte Blutfarbstoffabtrennund Nachweisschicht außerordentlich dünn ist. Bevorzugt liegt die mittlere Trockenschichtdicke unter 0,05 mm, besonders bevorzugt unter 0,025 mm, ganz besonders bevorzugt unter 0,015 mm.

Neben der einfachen Handhabung zeichnet sich der erfindungsgemäße Testträger durch eine extrem kurze Reaktionszeit und intensive Farbbildung aus. Außerdem ist das Testfeld selbstdosierend. Es nimmt aus einer im Überschuß (zum Beispiel zwischen 5 µl und 50 µl) aufgetragenen Probe nur eine reproduzierbare Teilmenge (zum Beispiel 2 µl) auf.

Die Erfindung stellt eine grundsätzliche Abkehr von den bisher vorgeschlagenen NW-Testfeldern dar. Während bei den bisherigen Versuchen das Testfeld meist aus einem Schichtverbund bestand, der getrennte Schichten oder Zonen enthielt, von denen eine der Abtrennung der Erythrozyten und eine andere dem Nachweis durch eine Farbbildungsreaktion diente, werden erfindungsgemäß beide Funktionen in einer einzigen homogen zusammengesetzten Schicht, nämlich der Blutfarbstoffabtrenn- und Nachweisschicht, zusammengefaßt. Bevorzugt besteht das Testfeld nur aus der Blutfarbstoffabtrenn- und Nachweisschicht. Da diese extrem dünn ist und nur wenig Flüssigkeit aufnimmt, kann mit einer extrem kleinen Blutprobe gearbeitet werden. In Ausnahmefällen kann jedoch die Blutfarbstoffabtrenn- und Nachweisschicht mit anderen Testschichten kombiniert werden, beispielsweise einer auf der Probenaufgabeseite vorgelagerten Schicht, die eine laterale Ausbreitung bewirkt (spreading layer) oder eine Reagenzschicht, welche Reagenzien enthält, die mit den in der Blutfarbstoffabtrenn- und Nachweisschicht enthaltenen Reagenzien nicht verträglich sind.

Die Blutfarbstoffabtrenn- und Nachweisschicht kann mit ihrer Nachweisseite auf einer durchsichtigen Trägerfolie angebracht sein. Gemäß einer besonders vorteilhaften Alternative ist sie jedoch in eine offenporige Faserverbundstruktur imprägniert.

Gemäß einer weiteren besonders bevorzugten Ausführungsform kann die Blutfarbstoffabtrenn- und Nachweisschicht auf einer mikroporösen Kunststoffschicht (Membran) durch unmittelbares Beschichten der Membran angebracht sein. Als besonders günstig hat sich dabei die Kombination der Blutfarbstoffabtrenn- und Nachweisschicht mit einer asymmetrischen Membran erwiesen, die ihrerseits Erythrozytenabtrenneigenschaften hat. Derartige Membranen werden in neuerer Zeit für verschiedene Filtrationszwecke angeboten. Ein Überblick ist beispielsweise dem Artikel "The Structure and Some Properties of Graded Highly Asymmetrical Porous Membranes" von W. Wrasidlo und K.J. Mysels, Journal of Parenteral Science and Technology, 1984, Seite 24 bis 31, zu entnehmen. Von besonderem Vorteil ist dabei die Tatsache, daß die Blutfarbstoffabtrenn- und Nachweisschicht nicht nur Blutkörperchen abtrennt, sondern auch eine wirksame Abtrennung des Blutfarbstoffes, soweit dieser in praktisch vorkommenden Konzentrationen vorliegt, bewirkt. Experimentell wurde festgestellt, daß die erfindungsgemäße Schicht geeignet ist, selbst Proben mit einem Hämolyseanteil von bis zu 0,5 % zu verarbeiten. Dieser Wert liegt mit gutem Sicherheitsabstand über den in der Praxis vorkommenden Hämolysewerten von maximal etwa 0,1 %. Wenn man aus Blut mit einem Hämolyseanteil von 0,5 % die Blutkörperchen vollständig abtrennt, ist das resultierende Serum immer noch stark rot gefärbt. Wenn man jedoch ein solches Serum von der Probenaufgabeseite her auf eine erfindungsgemäße Blutfarbstoffabtrenn- und Nachweisschicht aufgibt, wird der Blutfarbstoff so vollständig abgetrennt, daß die analytische Messung auf der Auswerteseite innerhalb der Meßgenauigkeit davon praktisch nicht beeinträchtigt wird.

Die Erfindung wird im folgenden anhand eines in den Figuren schematisch dargestellten Ausführungsbeispiels näher erläutert; es zeigen:
- Fig. 1: eine stark vergrößerte Prinzipdarstellung eines Querschnitts durch eine für die Erfindung geeignete Testfeldstruktur im nassen Zustand,
- Fig. 2: eine stark vergrößerte Prinzidarstellung eines Querschnitts eines erfindungsgemäßen Testträgers,
- Fig. 3: eine Aufsicht auf eine für die Erfindung geeignete Faserverbundstruktur nach einer elektronenmikroskopischen Aufnahme,
- Fig. 4 und Fig. 5: eine stark vergrößerte Prinzipdarstellungen eines Querschnittes alternativer Ausführungsformen von Testfeldern,
- Fig. 6: mehrere Spektren zu einem der Beispiele,
- Fig. 7: eine graphische Darstellung zur Erläuterung des Einflusses der Zusammensetzung der schichtbildenden Masse bei einem der Beispiele.

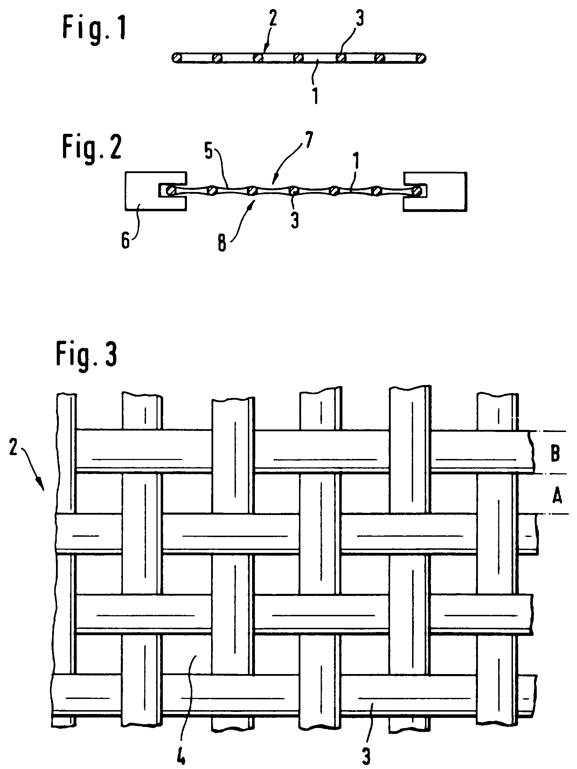

Figuren 1 und 2 zeigen im Querschnitt ein Testfeldmaterial, bei dem die Erythrozytenabrenn- und Nachweisschicht 1 in eine Faserverbundstruktur 2 imprägniert ist. Der Begriff "Faserverbundstruktur" bezeichnet dabei jeden Verbund von Fäden oder Fasern, der die für die Erfindung erforderlichen Eigenschaften, nämlich eine sehr geringe Dicke (maximal 0,1 mm, bevorzugt 0,02 bis 0,06 mm) und eine ausreichende Offenporigkeit, aufweist. Der Faserverbund kann vorzugsweise ein Gewebe oder ein Gewirke sein. Auch ein sehr dünnes und lockeres Vlies kann verwendet werden, ist jedoch weniger bevorzugt.

Die Faserverbundstruktur kann aus monofilen Fäden oder aus multifilen (d.h. aus vielen Einzelfäden bestehenden und gezwirnten) Fasern bestehen, wobei sich die monofilen Fäden besonders bewährt haben.

Fig. 3 zeigt die typische Struktur eines für die Erfindung geeigneten monofilen Gewebes. Die Fäden 3 haben eine lockere, gitterförmige Struktur mit offenen Poren 4. Die Porengröße A sollte zwischen 0,02 und 0,06 mm, vorzugsweise zwischen 0,03 und 0,04 mm liegen. Im dargestellten Fall quadratischer Poren bezeichnet der Begriff Porengröße deren Kantenlänge. Bei einer von der quadratischen Form abweichenden Porenform ist die Porengröße als Quadratwurzel aus dem mittleren Porenquerschnitt definiert.

Die Dicke B der Fäden, aus denen die Faserverbundstruktur gebildet ist, liegt vorzugsweise zwischen 0,02 und 0,06 mm, besonders bevorzugt zwischen 0,03 und 0,04 mm. Als Fadenmaterial hat sich Polyethylen bewährt. Es sind jedoch auch andere Materialien geeignet.

Wie erwähnt, besteht das Testfeld eines erfindungsgemäßen Testträgers vorzugsweise nur aus der auf einem Trägermaterial aufgebrachten Blutfarbstoffabtrenn- und Nachweisschicht. Ein solcher Testträger ist in Fig. 2 schematisch dargestellt. Das insgesamt mit 5 bezeichnete Testfeld wird von einem Rahmen 6 gehalten. Die Probe wird von der Probenaufgabeseite 7 her aufgegeben. Die optische Auswertung erfolgt auf der Nachweisseite 8.

Die dargestellte Struktur einer Blutfarbstoffabtrenn- und Nachweisschicht, welche in eine offenporige Faserverbundstruktur imprägniert ist, zeichnet sich vor allem dadurch aus, daß sie selbsttragend ist, d.h. es ist keine durchsichtige Trägerfolie als Unterlage erforderlich. Dabei ist jedoch wichtig, daß die Poren der Faserverbundstruktur so vollständig geschlossen sind, daß das auf der Probenaufgabeseite 7 aufgegebene Blut nicht durch kleine Spalten oder Risse auf die Nachweisseite 8 gelangen kann, weil durch die intensive Rotfärbung des Blutes schon relativ geringe Mengen das Ergebnis der optischen Messung verfälschen.

Um dies zu gewährleisten ist es vorteilhaft, wenn bei der Herstellung des Testfeldmaterials die Substanzen, aus denen die Blutfarbstoffabtrenn- und Nachweisschicht besteht, zu einer schichtbildenden Imprägniermasse verarbeitet werden, welche in eine Bahn aus Trägermaterial, die aus der offenporigen Faserverbundstruktur besteht, derartig imprägniert wird, daß die Imprägniermasse in die Poren des Trägermaterial weitgehend eindringt. Die Imprägniermasse wird dann mit Hilfe eines Abstreifwerkzeuges glattgestrichen. Danach wird die Testfeldmaterialbahn berührungsfrei getrocknet.

Insgesamt wird die Imprägniermasse so aufgetragen, daß die Hohlräume des Trägermaterials praktisch vollständig gefüllt und die Fäden beidseitig dünn mit Imprägniermasse bedeckt sind. Der Zustand nach dem Glattstreichen ist in Fig. 1 stark schematisiert dargestellt.

Durch den Trocknungsvorgang verringert sich die Schichtdicke der Imprägniermasse. Dadurch entsteht eine charakteristische Struktur, bei der die Oberfläche der Testschicht zwischen den Fäden leicht konkav gekrümmt ist, wie dies in Fig. 2 angedeutet ist.

Eine in eine Faserverbundstruktur imprägnierte Blutfarbstoffabtrenn- und Nachweisschicht, wie sie anhand der Figuren 1 bis 3 erläutert wurde, ist vor allem dann bevorzugt, wenn ein selbsttragendes Testschichtmaterial gewünscht wird, bei dem die Schicht beidseitig mit der Umgebungsluft in Kontakt steht. Dies gilt vor allem bei Reagenzsystemen, die Oxidationsschritte mit Hilfe des Luftsauerstoffs enthalten.

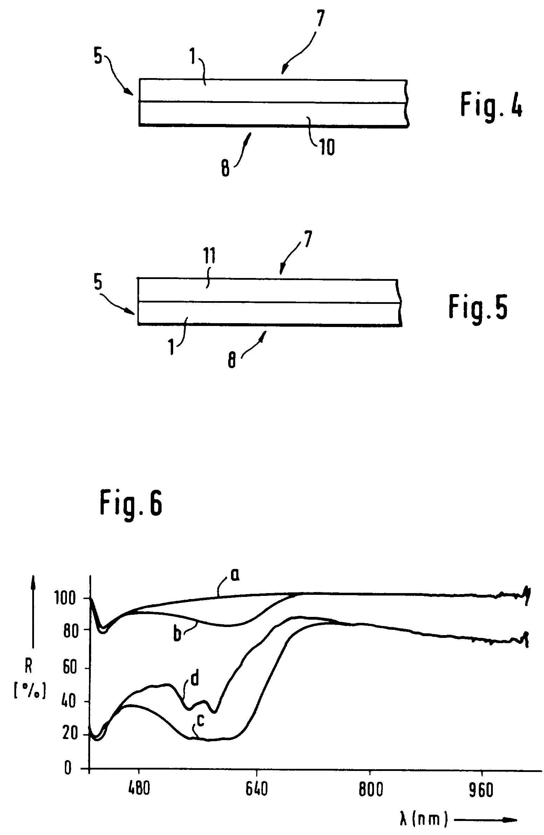

Figur 4 zeigt einen Aufbau des Testfeldes, wie er für ein Testsystem geeignet ist, welches unabhängig vom Luftsauerstoff funktioniert. Die Blutfarbstoffabtrenn- und Nachweisschicht 1 ist dabei auf eine durchsichtige Kunststoffolie 10 beschichtet.

Figur 5 zeigt die Ausführungsform, bei der die Blutfarbstoffabtrenn- und Nachweisschicht auf einer mikroporösen Kunststoffschicht (Membran) 11 beschichtetist. Die Membran ist vorzugsweise - wie dargestellt - der Blutaufgabeseite 7 zugewandt.

Die folgenden Beispiele dienen der weiteren Erläuterung der Erfindung.

### Beispiel 1:

Zur Herstellung einer Blutfarbstoffabtrenn- und Nachweisschicht zur quantitativen Analyse von Glucose aus Vollblut wird eine Dispersion folgender Zusammensetzung hergestellt:
Rezeptur:

| | Einwaage (g) | |
|---|---|---|
| | absolut | Feststoffgehalt |
| Polyvinylpropionat (PVP)-/Polyvinylacetat (PVA)-Dispersion (50%ig in Wasser) | 5 g | (2,5) |
| Titandioxid-Pulver in Wasser (Aufschlämmung 1:1) | 10 g | (5,0) |
| Methylvinylethermaleinsäureanhydrid-Copolymer (Gantrez AN 139; 5%ig in Wasser) | 8 g | (0,4) |
| Kaliumdihydrogenphosphat | 0,27 g | (0,3) |
| Dinatriumhydrogenphosphat-dihydrat | 0,28 g | (0,3) |
| Glucoseoxidase | 30 KU | (0,3) |
| Peroxidase | 500 KU | (0,15) |
| Methylaminobenzthiazolinon-hydrazon (MBTH) | 0,2 g | (0,2) |
| 3-Dimethylamino-benzosesäure | 0,2 g | (0,2) |
| Wasser dest. | 4,0 g | |
| Natriumhydroxid | 0,1 g | (0,1) |

Diese Mischung wird auf ein monofiles Polyethylen- oder Nylon-Gewebe von ca. 0,05 mm Dicke mit Hilfe eines Rakels (Rakelspalt ca. 0,08 mm) aufgebracht und unmittelbar anschließend mit Warmluft bei 60° C ca. 30 Minuten berührungsfrei getrocknet.

Die so hergestellte Reagenzschicht zeigt mit glucosehaltigem Blut in Abhängigkeit von der Glucosekonzentration gut abgestufte blaue Reaktionsfarben, welche mit einem Remissionsphotometer bei 565 nm vermessen werden können. Eicht man das Meßgerät mit dem Leerwert der Reaktions schicht auf 100% Remission und mit einer schwarzen Folie auf 4% Remission, werden für die entsprechenden Glucosekonzentrationen im Blut folgende Remissionen erhalten:

| Glucose in Blut mg/dl | |
|---|---|
| 0 | 100% |
| 40 | 81% |
| 80 | 67% |
| 100 | 62% |
| 300 | 39% |
| 400 | 34% |
| 500 | 30% |
| 600 | 26% |

In Figur 6 sind vier verschiedene Spektren von remissionsphotometrischen Messungen dargestellt, bei denen die Remission (in %) gegen die Wellenlänge (in Nanometer) dargestellt ist. Die Spektren a, b und c beziehen sich auf Testfelder gemäß Beispiel 1 mit einer glucosefreien Probe (a), einem Glucosegehalt von 30 mg/dl (b) und einem Glucosegehalt von 700 mg/dl (c). Man erkennt, das typische Absorptionsminimum des Indikators MBTH. Die Differenz zwischen dem Spektrum a und den Spektren b beziehungsweise c bei der gewählten Meßwellenlänge stellt das Nutzsignal dar.

Spektrum d betrifft einen Fall, bei dem die Probe wie bei Beispiel b 30 mg/dl Glucose enthält, jedoch mit einer Nachweisschicht gearbeitet wird, bei der die Blutfarbstoffabtrennung unvollständig ist. Der rote Blutfarbstoff führt zu einem ausgeprägtem Minimum in dem für die Messung wichtigen Bereich. Es ist offensichtlich, daß hierdurch eine nicht tolerierbare Verfälschung des Meßsignals resultiert.

### Beispiel 2:

Um den Einfluß verschiedener Konzentrationen an Pigment und Filmbildner auf die Blutfarbstoffabtrenneigenschaft zu testen, wurde die Rezeptur von Beispiel 1 hinsichtlich des Gehaltes dieser beiden Komponenten variiert.

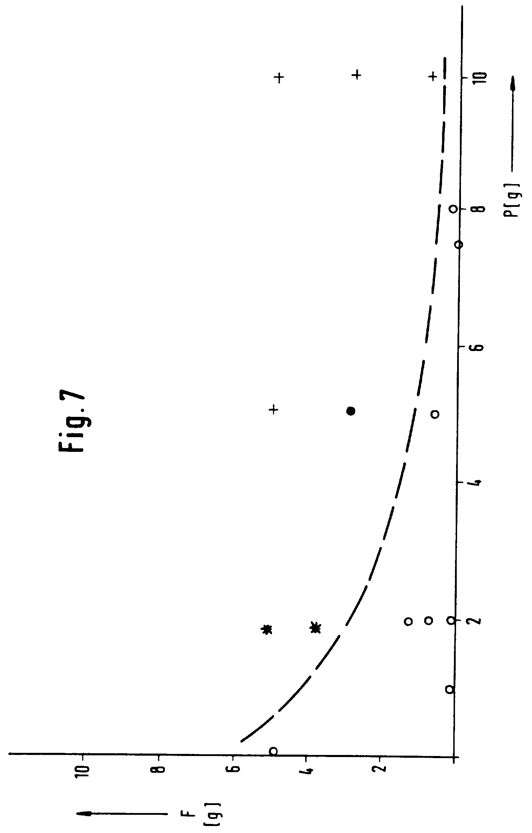

Die Ergebnisse sind in Figur 7 dargestellt, wobei auf der Abszisse das Feststoffgewicht des Pigments P und auf der Ordinate das Feststoffgewicht des Filmbildners F aufgetragen ist. Die offenen Kreise bezeichnen Zusammensetzungen, bei denen keine befriedigende Blutfarbstoffabtrenneigenschaft erreicht wurde, während die Pluszeichen Zusammensetzungen mit befriedigender Funktion bezeichnen. Die Sternzeichen bezeichnen Zusammensetzungen, bei denen zwar eine ausreichende Abtrennung von Erythrozyten erreicht wird, die strahlungsblockierenden Eigenschaften der Schicht jedoch unzureichend sind. Das Wertepaar des Beispiels 1 ist durch ein eingekreistes Pluszeichen gekennzeichnet. Es zeichnet sich durch eine besonders gute Blutfarbstoff-Abtrennwirkung aus.

Die Ergebnisse zeigen, daß keine gute Abtrennwirkung erreicht wird, wenn sowohl die eingesetzte Menge Pigment als auch die eingesetzte Menge Filmbildner sehr klein ist. Ein befriedigendes Ergebnis kann sowohl durch Erhöhung des Pigmentanteils als auch durch Erhöhung des Filmbildneranteils erreicht werden. Besonders gute Ergebnisse werden erreicht, wenn beide in einem etwa ausgewogenen Verhältnis zueinander stehen und ihre Gesamtmenge in der schichtbildenden Rezeptur ausreichend hoch ist.

### Beispiel 2a:

Um den Einfluß verschiedener Quellmittel zu dokumentieren, wurde in der Rezeptur von Beispiel 1 das Gantrez AN 139 ersetzt durch Keltrol F (Hersteller: Kelco/Ail International GmbH, Hamburg, Deutschland). Die nachfolgende Tabelle zeigt für beide Rezepturen den Verlauf der gemessenen diffusen Reflexion (% Rem) für zwei verschiedene Blutproben. Man erkennt, daß bei Verwendung des Quellmittels Gantrez AN 139 sich bereits nach 30 Sekunden ein stabiler Wert einstellt, der sich in den folgenden 30 Sekunden nicht ändert. Bei Verwendung von Keltrol F ist die Annäherung an den Endwert langsamer. Auch hier ist jedoch bei geeigneter Eichung eine zuverlässige Messung nach weniger als einer Minute mit guter Genauigkeit möglich.

| | Zeit seit Probenaufgabe | Blut 1 | Blut 2 |
|---|---|---|---|
| | (sec) | (%) Rem. | |
| Keltrol F | 10 | 52.8 | 36.7 |
| | 20 | 47.2 | 23.1 |
| | 30 | 45.9 | 18.7 |
| | 40 | 44.8 | 17.2 |
| | 50 | 44.2 | 16.5 |
| | 60 | 44.0 | 16.3 |
| | | | |
| Gantrez AN 139 | 10 | 45.4 | 22.2 |
| | 20 | 44.6 | 17.6 |
| | 30 | 44.1 | 16.9 |
| | 40 | 44.0 | 16.2 |
| | 50 | 44.0 | 16.2 |
| | 60 | 44.0 | 16.2 |

Mit weniger gut quellenden Quellmitteln, beispielsweise dem vielfach in Testschichtrezepturen eingesetzten Alginat, werden keine vergleichbar guten Ergebnisse erreicht.

### Beispiel 3:

Herstellung eines Testfeldes für einen Testträger zum Nachweis von Triglyceriden in Blut.
Rezeptur:

| | Einwaage (g) | |
|---|---|---|
| | absolut | Feststoffgehalt |
| Polyvinylpropionat-Dispersion (50%ig in Wasser) | 7 g | (3,5) |
| Titandioxid | 4 g | (4,0) |
| Keltrol F (0,5 %ig in H₂O) | 27 g | (0,15) |
| Adenosin-5'-triphosphat,di-Natriumsalz | 60 mg | (0,06) |
| Magnesiumsulfat-7-hydrat | 60 mg | (0,06) |
| Dioctylnatriumsulfosuccinat | 100 mg | (0,1) |
| 1-(4-Methylphenyl)-semicarbazid | 1,5 mg | (0,002) |
| Detergens Triton® X-100 | 200 mg | (0,2) |
| Cholesterinesterase | 3 KU | (0,25) |
| Glycerinphosphatoxidase | 0,8 KU | (0,01) |
| Glycerokinase | 3 KU | (0,15) |
| Peroxidase | 500 MU | (0,15) |
| 4(5)-(4-Dimethylamino-phenyl)-2-(4-hydroxy-3,5-dimethoxyphenyl)-5(4)-methylimidazol x HCl | 50 mg | (0,05) |
| Phosphatpuffer 0,2M / pH7,5 | 4 g | (0,05) |

Aus den Rezepturkomponenten wird eine homogene Beschichtungsmasse hergestellt. Der pH-Wert wird kontrolliert und erforderlichenfalls auf pH 7,5 korrigiert. Unmittelbar danach wird die Masse in ein multifiles Polyethylengewebe (Dicke ca. 0,04 mm) in einer Naßfilmdicke von ca. 0,06 mm gerakelt und bei 50° C in einem Umlufttrockenschrank ca. 60 Minuten getrocknet.

Bei der Analyse von Triglycerid-haltigen Blutproben werden gut abgestufte Blaufärbungen im Konzentrationsbereich 50 bis 300 mg/dl erhalten, die bei 660 nm remissionsphotometrisch ausgewertet werden können.

### Beispiel 4:

Um die Brauchbarkeit verschiedener Pigment-Substanzen zu testen wurde die Rezeptur von Beispiel 1 hinsichtlich des Pigments abgewandelt und Versuche mit verschiedenen Konzentrationsverhältnissen gemacht. In der folgenden Tabelle sind die Trockensubstanz-Gewichte von Rezepturen angegeben, die erprobt wurden und zufriedenstellende Blutfarbstoffabtrenneigenschaften aufweisen:

| Polyvinylpropionat | Bariumsulfat |
|---|---|
| 5 g | 10 g |
| 5 g | 1 g |

| Polyvinylpropionat | Orgasol 2002 |
|---|---|
| 5 g | 10 g |
| 5 g | 1 g |

Das Bariumsulfat hatte eine mittlere Teilchengröße von 0,6 µm (Hersteller: Merck, Darmstadt, Deutschland).

Orgasol 2002 ist ein Polyamid-Partikelpulver mit Pigmenteigenschaften, welches von der Firma Atochem, Elf-Aquitaine, Ortez, Frankreich, hergestellt wird. Die mittlere Teilchengröße liegt bei 0,4 µm.

### Beispiel 5:

Herstellung eines Reagenzfilms zum reduktiven Nachweis von Glucose.
Rezeptur:

| | Einwaage (g) | |
|---|---|---|
| | absolut | Feststoffgehalt |
| KeltrolF (Hersteller: Kelco/Ail International GmbH, Hamburg, Deutschland) 2%ig gelöst in Citratpuffer 0,5 M/pH 7 | 20 g | (0,4) |
| Titandioxid in Citratpuffer 0,5M/pH (Aufschlämmung 1:1) | 40 g | (20,0) |
| Polyvinylpropionat (PVP)-/Polyvinylacetat (PVA) (Dispersion 50%ig in Wasser) | 5 g | (2,5) |
| Natriumnonylsulfatlösung (15%ig in Wasser) | 3 g | (0,4) |
| Tartrazin (Hersteller: Merck) | 2 g | (2,0) |
| Tetraethylammoniumchlorid | 7 g | (7,0) |
| 2,18-Phosphormolybdaensäure | 16 g | (16,0) |
| Kollidon 25 (Hersteller: BASF, Ludwigshafen, Deutschland) | 2 g | (2,0) |
| N,N-Bis-hydrxyethyl-p-nitroso-anilin | 140 mg | (0,140) |
| GOD | 2 g | (2,0) |
| Wasser | 40 g | - |

Die Rezeptur wird homogenisiert und auf eine Folie aus Pokalon (Hersteller: Lonza, Schweiz) in einer Dicke von 0,1 mm aufgerakelt. Anschließend wird bei 60° C getrocknet. Der erhaltene Reagenzfilm zeigt ausgezeichnete Erythrozyten-Abtrenneigenschaften und eine intensive Farbbildung in Abhängigkeit von der Glucosekonzentration.

Dieses Beispiel zeigt, daß sich eine erfindungsgemäße Blutfarbstoffabtrenn- und Nachweisschicht auch in Fällen herstellen läßt, bei denen aufgrund spezifischer Besonderheiten des Tests die löslichen Bestandteile des Reagenzsystems in verhältnismäßig hoher Konzentration vorhanden sein müssen. Im vorliegenden Fall liegt das summierte Trockengewicht der Indikatorkombination aus Tetraethylammoniumchlorid und 2,18-Phosphormolybdänsäure etwa in der gleichen Größenordnung wie das summierte Trockengewicht von Pigment und Filmbildner.

## Patentansprüche

1. Testträger zur Bestimmung eines Analyten aus Vollblut mit Hilfe eines in dem Testträger enthaltenen Reagenzsystems, welches ein Farbbildungsreagenz einschließt, mit einem Testfeld (5),
welches eine Probenaufgabeseite (7), auf der die Blutprobe zugeführt wird und eine Nachweisseite (8), auf der infolge der Reaktion des Analyten mit dem Reagenzsystem eine optisch nachweisbare Veränderung stattfindet, aufweist, und
welches so ausgebildet ist, daß in der Probe enthaltende Erythrozyten nicht auf die Nachweisseite gelangen, wobei
das Testfeld (5) eine Filmschicht als kombinierte Blutfarbstoffabtrenn- und Nachweisschicht (1) aufweist, welche
aus einer Dispersion oder einer Emulsion eines polymeren Filmbildners gebildet ist, die in homogener Verteilung ein Pigment in einer Konzentration von mindestens 30 Gew-%, bezogen auf den Feststoffgehalt der filmbildenden Masse, den polymeren Filmbildner, das Farbbildungsreagenz und ein Quellmittel enthält,
wobei die Quellfähigkeit des Quellmittels so hoch ist, daß die optisch nachweisbare Veränderung auf der Nachweisseite nach maximal einer Minute meßbar ist.

2. Testträger nach einem der vorhergehenden Ansprüche, **dadurch gekennzeichnet,** daß die mittlere Dicke der Blutfarbstoffabtrenn- und Nachweisschicht (1) maximal 0,05 mm, bevorzugt maximal 0,025 mm, besonders bevorzugt maximal 0,015 mm, beträgt.

3. Testträger nach einem der vorhergehenden Ansprüche, **dadurch gekennzeichnet,** daß die Blutfarbstoffabtrennund Nachweisschicht (1) in eine offenporige Faserverbundstruktur (2) derartig imprägniert ist, daß sie deren Poren überspannt und somit schließt.

4. Testträger nach Anspruch 3, **dadurch gekennzeichnet,** daß die mittlere Porengröße der Faserverbundstruktur (2) zwischen 0,02 und 0,06 mm, vorzugsweise zwischen 0,03 und 0,05 mm beträgt.

5. Testträger nach Anspruch 1 oder 2, **dadurch gekennzeichnet,** daß die Blutfarbstoffabtrenn- und Nachweisschicht (1) auf eine mikroporöse Kunststoffschicht (11) beschichtet ist.

6. Testträger nach Anspruch 5, **dadurch gekennzeichnet,** daß die mikroporöse Kunststoffolie (11) der Probenaufgabeseite (7) des Testträgers zugewandt ist.

7. Testträger nach einem der Ansprüche 5 oder 6, **dadurch gekennzeichnet,** daß die mikroporöse Kunststoffolie (11) eine asymmetrische Struktur hat und Erythrozyten zumindest teilweise abtrennt.

8. Testträger nach Anspruch 1 oder 2, **dadurch gekennzeichnet,** daß die Blutfarbstoffabtrenn- und Nachweisschicht (1) auf eine Kunststoffolie (10) beschichtet ist.

## Claims

1. Test carrier for the determination of an analyte from whole blood with the aid of a reagent system contained in the test carrier, said reagent system comprising a colour formation reagent, with a test field (5),
which has a sample application side (7), to which the blood sample is fed, and a detection side (8) on which, as a result of reaction of the analyte with the reagent system, an optically detectable change occurs, and
which is designed so that erythrocytes contained in the sample do not gain access to the detection side,
the test field (5) having a film layer as combined blood pigment separation and detection layer (1), which
is formed from a dispersion or emulsion of a polymeric film former, said dispersion or emulsion containing, in homogeneous distribution, a pigment in a concentration of at least 30% by weight relative to the solid substance content of the film-forming mass, the polymeric film former, the colour formation reagent and a swelling agent,
the swelling properties of the swelling agent being so good that the optically detectable change on the detection side is measurable after a maximum of one minute.

2. Test carrier according to one of the preceding claims, **characterised in that** the mean thickness of the blood pigment separation and detection layer (1) is at most 0.05 mm, preferably at most 0.025 mm, especially preferably at most 0.015 mm.

3. Test carrier according to one of the preceding claims, **characterised in that** the blood pigment separation and detection layer (1) is impregnated into an open-pore composite fibre structure (2) so that it spans, and thus closes, its pores.

4. Test carrier according to claim 3, **characterised in that** the mean pore size of the composite fibre structure (2) is between 0.02 and 0.06 mm, preferably between 0.03 and 0.05 mm.

5. Test carrier according to claim 1 or 2, **characterised in that** the blood pigment separation and detection layer (1) is coated on to a microporous plastic layer (11).

6. Test carrier according to claim 5, **characterised in that** the microporous plastic film (11) faces the sample application side (7) of the test carrier.

7. Test carrier according to one of claims 5 or 6, **characterised in that** the microporous plastic film (11) has an asymmetrical structure, and at least partially separates erythrocytes.

8. Test carrier according to claim 1 or 2, **characterised in that** the blood pigment separation and detection layer (1) is coated on to a plastic film (10).

## Revendications

1. Support de test pour la détermination d'un analyte dans le sang total, à l'aide d'un système de réactifs contenu dans un support de test, qui comprend un réactif chromogène, avec une zone de test (5),
qui présente une face d'application de l'échantillon (7), sur laquelle est appliquée l'échantillon de sang et une face de détection (8), sur laquelle, par suite de la réaction de l'analyte avec le système de réactifs, il se produit une variation optiquement détectable, et
qui est conçu de façon que les érythrocytes contenus dans l'échantillon ne parviennent pas sur la face de détection,
la zone de test (5) présentant une couche de film en tant que couche combinée de séparation des colorants du sang et de détection (1), qui
est constituée d'une dispersion ou d'une émulsion d'un agent filmogène polymère, qui contient, en dispersion homogène, un pigment en une concentration d'au moins 30 % en poids, par rapport à la teneur en substances solides de la matière filmogène, l'agent filmogène polymère, le réactif chromogène et un agent gonflant,
la capacité d'expansion de l'agent gonflant étant suffisamment élevée pour que la variation optiquement détectable puisse être mesurée sur la face de détection après une minute au maximum.

2. Support de test selon l'une quelconque des revendications précédentes, caractérisé par le fait que l'épaisseur moyenne de la couche de séparation des colorants du sang et de détection (1) est au maximum de 0,05 mm, de préférence au maximum de 0,025 mm et en particulier, au maximum de 0,015 mm.

3. Support de test selon l'une quelconque des revendications précédentes, caractérisé par le fait que la couche de séparation des colorants du sang et de détection (1) imprègne une structure de fibres composite (2) à pores ouverts de façon que les pores de celle-ci soient recouverts, et donc fermés.

4. Support de test selon la revendication 3, caractérisé par le fait que la taille moyenne des particules de la structure de fibres composite (2) est comprise entre 0,02 et 0,06 mm, de préférence entre 0,03 et 0,05 mm.

5. Support de test selon la revendication 1 ou 2, caractérisé par le fait que la couche de séparation des colorants du sang et de détection (1) est appliquée sur une couche de matière plastique microporeuse (11).

6. Support de test selon la revendication 5, caractérisé par le fait que la feuille de matière plastique microporeuse (11) est tournée vers la face d'application de l'échantillon (7) du support de test.

7. Support de test selon l'une des revendications 5 ou 6, caractérisé par le fait que la feuille de matière plastique microporeuse (11) présente une structure asymétrique et sépare au moins partiellement les érythrocytes.

8. Support de test selon l'une des revendications 1 ou 2, caractérisé par le fait que la couche de séparation des colorants du sang et de détection (1) est appliquée sur une feuille en matière plastique (10).
